# FASCICULE DE BREVET EUROPEEN

(11) **EP 0 684 794 B1**
(45) Date de publication et mention de la délivrance du brevet: **25.11.1998**
(21) Numéro de dépôt: 95905159.0
(22) Date de dépôt: 20.12.1994
(51) Int. Cl.: A61B 17/58

(54) **CLOU TIBIAL CENTRO-MEDULLAIRE ET SON APPAREIL DE POSE**
TIBIA-MARKNAGEL UND VORRICHTUNG ZUM SETZEN
CENTROMEDULLARY TIBIAL NAIL AND APPARATUS FOR INSERTION THEREOF

(30) Priorité: 21.12.1993 FR 9315837
(43) Date de publication de la demande: 06.12.1995
(73) Titulaire: MEDINOV SA, F-42312 Roanne Cedex (FR)
(72) Inventeur: CATON, Jacques, Allée Féraud, F-69300 Caluire (FR); TROUILLOUD, Pierre, F-21000 Dijon (FR); NACHIN, Patrick, F-25550 Dung (FR)
(74) Mandataire: Moncheny, Michel
(86) Numéro de dépôt international: FR9401493
(87) Numéro de publication internationale: WO9517857

(56) Documents cités:
- DE-A- 3 244 243
- FR-A- 2 647 006
- US-A- 5 248 313

## Description

Plus particulièrement, l'invention concerne un clou centro-médullaire destiné à être impacté dans le canal tibial.

On rappelle que les clous orthopédiques peuvent être constitués par des tiges longues généralement creuses, que l'on introduit dans le canal médullaire d'un os long afin de réduire une fracture, corriger un défaut d'axe ou d'angulation, allonger un membre......

On sait, par ailleurs, que l'anatomie de la jambe est très différente de celle de la cuisse de sorte que dans l'un et l'autre cas les clous seront de conception différente. La section transversale du tibia, dans sa partie proximale, a une forme générale s'inscrivant très sensiblement dans un triangle dont les sommets sont arrondis.

Compte tenu des fonctions spécifiques susceptibles d'être remplies par le cou centro-médullaire, il est particulièrement important que le cou soit totalement immobilisé et parfaitement table pendant toute la durée de mise en place sur le patient, et ce en dépit de différents mouvements et de différentes sollicitations que celui-ci peut provoquer pendant son activité journalière A défaut, il peut en résulter des micro mouvements préjudiciables à une bonne reformation osseuse, de tels micro-mouvements pouvant même s'amplifier nécessitant alors une intervention chirurgicale de reprise. Ce problème de fixation est d'autant plus important que le tibia est un os très peu corticalisé dans sa moitié supérieure.

Il ressort des techniques actuellement utilisées qu'un clou centro-medullaire tibial est immobilisé par une ou des vis placées dans la partie proximale et dans la partie distale, par tous moyens connus et appropriés.

Il apparait que les deux vis disposées dans la partie proximale du cou sont toujours placées dans les plans de référence constitués par le plan frontal ou le plan sagittal (par exemple FR-A-2 647 006). Si la fixation distale donne satisfaction, il n'en n'est pas de même pour la fixation proximale compte tenu des sollicitations mécaniques de toutes sortes et de toutes origines situées à ce niveau, et de sa très faible corticalisation.

Généralement, la partie proximale du cou est fixée par au moins une vis introduite dans la partie avant du tibia qui est la plus accessible lors de l'opération chirurgicale, mais qui constitue la zone anguleuse de la section tibiale. Or cette zone, même si elle est très légèrement arrondie, ne se prête pas particulièrement à une attaque nette et franche, c'est-à-dire sans glissement d'un foret de perçage par exemple. En outre, la ou les vis coopèrant avec un minimum de surface osseuse , l'ancrage n'est pas assuré d'une manière sûre et efficace, tandis que l'os se trouve fragilisé.

Ainsi, non seulement le chirurgien éprouve à cet endroit des difficultés pour réaliser correctement et aisément son travail, mais en outre, il ne peut en toute assurance, réaliser celui-ci dans de bonnes conditions. S'il ajoute une deuxième vis perpendiculairement à la première, le système de visée est encombrant et le percage doit être réalisé sur une face oblique.

L'invention s'est fixée pour but de remédier à ces inconvénients de manière simple, sûre, efficace et rationnelle.

Le problème que se propose de résoudre l'invention est d'assurer une immobilisation totale et fiable d'un cou centro-medullaire en utilisant avantageusement l'anatomie du tibia à savoir les deux faces planes symétriques ou sensiblement symétriques par rapport au plan vertical défini par la zone antérieure légèrement arrondie et le centre du tibia et en utilisant la présence d'une zone corticalisée de solidité satisfaisante dans les deux angles postérieurs opposés aux deux faces planes précitées.

Pour résoudre un tel problème, les trous situés dans la partie proximale du clou et destinés à recevoir les organes de fixations sont orientés pour être disposés, après impaction du cou dans le canal centro -médullaire, en alignement co-axial avec les organes engagés très sensiblement perpendiculairement aux surfaces osseuses planes de la section tibiale de forme générale très sensiblement triangulaire .
Un tel clou est décrit dans la revendication 1, son appareil de pose dans la revendication 2.

Les trous sont orientés de l'ordre de 45° par rapport aux plans frontal et sagittal du tibia, et sont décalés de 90° et situés dans deux plans hoziontaux différents.

Ainsi, gràce à ce double blocage de la tige du cou dans le tibia, on empêche totalement tous micro-mouvements. En utilisant les faces planes du tibia, le chirurgien acquiert, de plus, une plus grande facilité de perçage et de pose des vis qui, chacune traversant le cou, ont leur extrémité placée en appui mécanique sur la corticale de la zone angulaire opposée, de sorte que chaque vis repose sur les corticales par leurs deux extrémités répartissant ainsi les efforts mécaniques exercés Si les vis sont munies d'une tête plate, celles-ci s'appuient correctement sur les faces osseuses, sinon les vis affleurent parfaitement ce qui est très important pour le patient, puisque, dans cette région de la jambe, le tibia est juste sous la peau et que de ce fait il est fréquent de déplorer des infections causées par des vis dont l'extrémité a traversé la peau.

Le clou tel que défini selon les caractéristiques de l'invention, est mis en place au moyen d'un appareil qui comprend des agencements pour le montage d'un cadre de visée et de perçage équipé de moyens aptes à assurer l'orientation et le guidage d'un outil de perçage d'une manière coaxiale aux trous formés dans la partie proximale du clou.

Avantageusement, les moyens sont constitués par des douilles formées angulairement à 45° par rapport au plan vertical.

Les douilles sont décalées latéralement pour correspondre aux trous que présente la partie proximale du clou.

L'appareil viseur impacteur tel que défini convient aussi bien pour le côté gauche que pour le côté droit, sans système amovible d'adaptation, aucun trou n'étant situé à 90°.

L'invention est exposée, ci-après plus en détail à l'aide des dessins annexés, dans lesquels :
La figure 1 est une vue de face du clou selon l'invention.
La figure 2 est une vue en plan correspondant à la figure 1.
La figure 3 est une vue en perspective montrant l'appareil impacteur viseur pour la mise en place du clou selon l'invention.
La figure 4 est une vue en coupe montrant le positionnement de l'appareil pour la mise en place des vis, de la partie proximale du clou impacté dans le canal médullaire du tibia.
La figure 5 est une vue partielle en plan de l'appareil viseur.

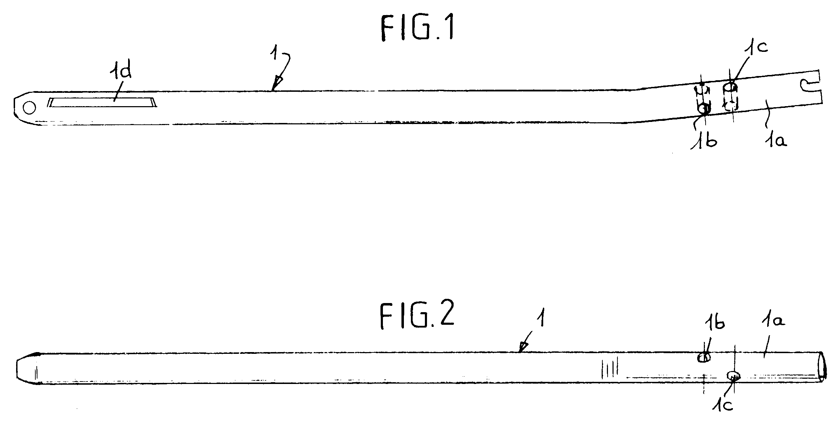

Comme le montrent les figures 1 et 2, le clou tibial centro médullaire est réalisé à partir d'une tige creuse (1), dont la partie proximale (1a) est cintrée pour correspondre au profil anatomique du tibia. Cette partie (1a) présente des trous (1b) (1c) pour l'engagement de vis de fixation (2).

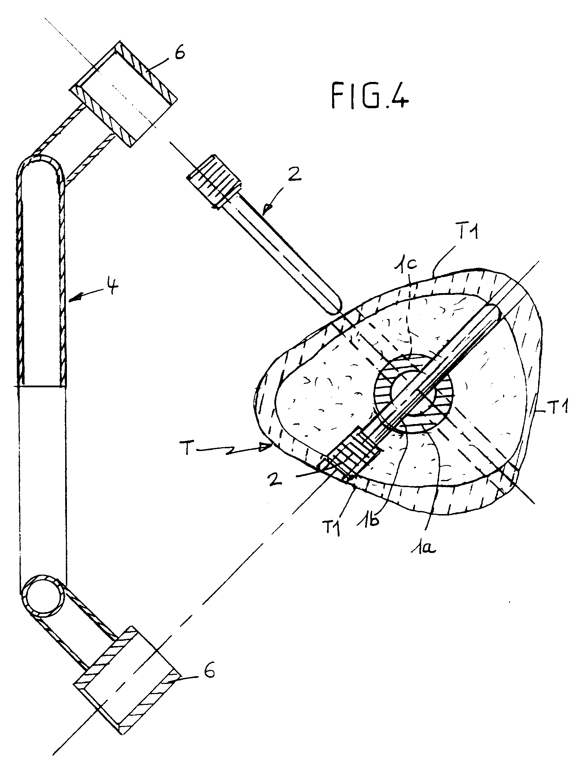

Selon l'invention, les trous (1b) (1c) sont orientés pour être disposées en alignement coaxial avec les vis (2) qui sont engagées très sensiblement perpendiculairement aux surfaces osseuses planes (T1) de la section tibiale (T) (figure 4). Les trous (1b) (1c) sont orientés très sensiblement de 45° par rapport aux plans frontal et sagittal du tibia. De même, ces trous (1b) et (1c) sont décalés en hauteur de 90° en étant situés dans deux plans horizontaux différents.

De manière connue, le clou (1) tel que défini, peut présenter au niveau de sa partie distale, deux fentes diamétralement opposées (1d) pour le débordement d'ailettes que présente une tige, non illustrée, introduite dans l'alésage du clou centro médullaire.

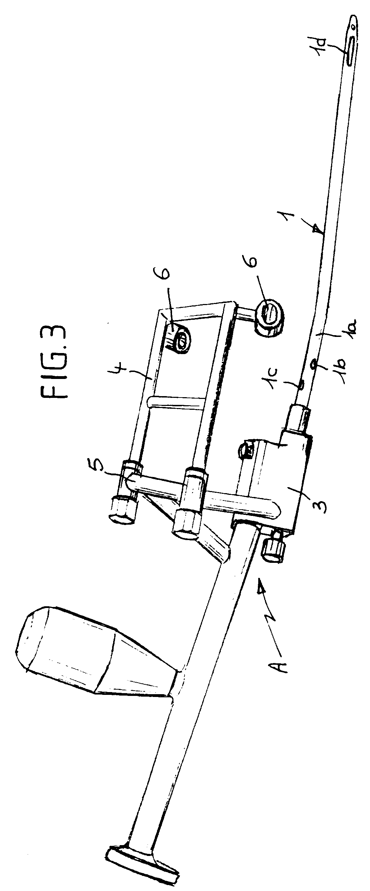

Compte-tenu des caractéristiques du clou et notamment de l'orientation spécifique des trous (1b) (1c) situés en partie proximale il a été concu et mis au point un appareil impacteur viseur (figures 3, 4 et 5). La conception générale de cet appareil, désigné dans son ensemble par (A), n'est pas décrite en détail car parfaitement connue par un homme du métier.

On rappelle simplement que cet appareil comprend un corps (3) agencé pour permettre l'accouplement et l'orientation angulaire du chou tibial (1). Ces agencements sont par exemple conformes à ceux définis dans le brevet FR-A-2689751 dont le demandeur de la présente est également titulaire.

Selon l'invention, l'appareil (A) comprend des agencements pour le montage d'un cadre de visée et de perçage (4). Ce cadre (4) est fixé d'une manière démontable, en bout d'un élément support (5) solidaire du corps (3). L'extrémité libre du cadre (4) présente des douilles (6) aptes à assurer l'orientation d'un outil de perçage, d'une manière coaxiale aux trous (1b) (1c) de la partie proximale du clou (2). Ces douilles (6) sont donc formées angulairement à 45° par rapport au plan vertical (figure 4).

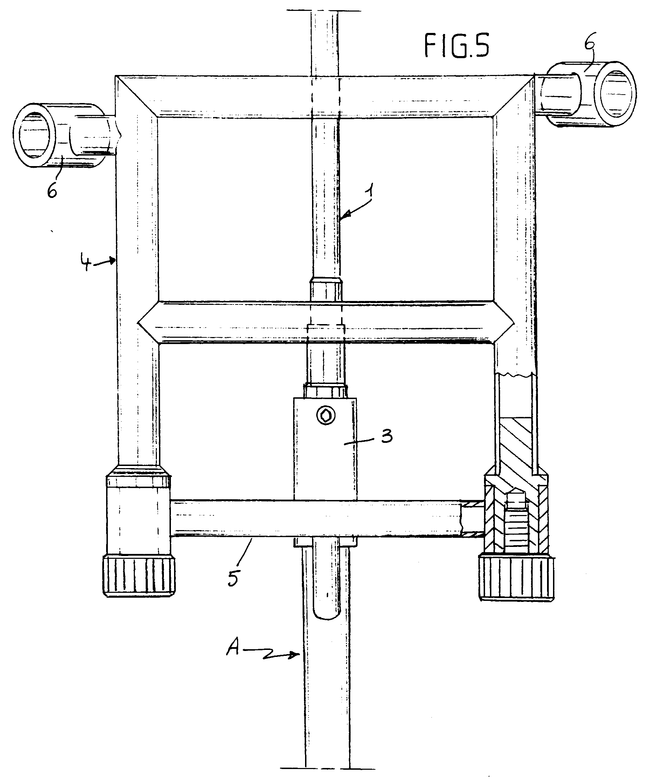

De la même façon que les trous (1b) et (1c), les douilles (6) sont décalées latéralement (figure 5).

Les caractéristiques du clou tel que défini, s'appliquent à tous types de clous tibiaux, notamment à des clous d'allongement.

Les avantages ressortent bien de la description, en particulier on souligne et on rappelle :
- les vis coopèrent avec les parties planes ou sensiblement planes de la section transversale du tibia, là ou la surface osseuse est la plus importante
- la facilité de perçage,
- le respect de l'anatomie,
- l'amélioration de l'ancrage à obtenir en empêchant tout micro-mouvements.

## Revendications

1. Clou tibial centro-médullaire présentant dans une partie proximale cintrée (1a) des trous (1b) (1c) pour le passage d'organes de fixation (2), caractérisé en ce que les trous (1b) (1c) sont ménagés perpendiculairement à l'axe du clou et orientés de 45 degrés environ par rapport à un plan de symétrie de clou, et en ce que les trous (1b) (1c) sont décalés de 90 degrés et situés dans deux plans transversaux différents du clou, perpendiculaires à son axe.

2. Appareil pour la pose d'un clou selon la revendication 1, présentant des moyens pour l'accouplement coaxial et l'orientation angulaire du cou (1), caractérisé en ce que ledit appareil comprend un cadre de visée et de perçage (4) équipé de douilles (6) aptes à assurer l'orientation et le guidage d'un outil de perçage d'une manière coaxiale aux trous (1b) (1c) formés dans la partie proximale (1a) du clou (1), ces douilles étant formées angulairement à 45 degrés par rapport à un plan vertical, et décalées latéralement pour correspondre aux trous (1b, 1c) de la partie proximale (1a) du clou (1).

## Claims

1. A centro-medullary tibial nail having in a bent proximal portion (1a) holes (1b) (1c) for the passing of fixing elements (2), characterized in that the holes (1b) (1c) are arranged perpendicularly to the axis of the nail and orientated at approximately 45 degrees relative to a plane of symmetry of the nail, and in that the holes (1b) (1c) are offset by 90 degrees and situated in two different transverse planes of the nail, perpendicular to its axis.

2. An apparatus for fitting a nail in accordance with claim 1, having means for the coaxial fastening and angular orientation of the nail (1), characterized in that the said apparatus comprises a pointing and drilling frame (4) provided with sockets (6) capable of ensuring the orientation and guidance of a drilling tool coaxially with the holes (1b) (1c) formed in the proximal portion (1a) of the nail (1), these sockets being formed angularly at 45 degrees relative to a vertical plane and laterally offset to correspond to the holes (1b) (1c) of the proximal portion (1a) of the nail (1).

## Patentansprüche

1. Tibia-Marknagel, der in einen gebogenen proximalen Teil (1a) Löcher (1b) (1c) für den Durchgang von Befestigungselementen (2) aufweist, dadurch gekennzeichnet, daß die Löcher (1b) (1c) senkrecht auf die Achse des Nagels ausgespart und ungefähr 45 Grad zu einer Symmetrieebene des Nagels ausgerichtet sind, und daß die Löcher (1b) (1c) um 90 Grad versetzt sind und in zwei unterschiedlichen Transversalebenen des Nagels senkrecht auf seine Achse angeordnet sind.

2. Vorrichtung zum Setzen eines Nagels nach Anspruch 1, die Mittel zur koaxialen Kopplung und winkelmäßigen Ausrichtung des Nagels (1) umfaßt, dadurch gekennzeichnet, daß die Vorrichtung einen Visier- und Bohrrahmen (4) umfaßt, der mit Hülsen (6) versehen ist, die die Ausrichtung und die Führung eines Bohrwerkzeugs auf koaxiale Weise zu den Löchern (1b) (1c), die in dem proximalen Teil (1a) des Nagels (1) ausgebildet sind, gewährleisten können, wobei diese Hülsen in einem Winkel von 45 Grad zu einer Vertikalebene ausgebildet und seitlich versetzt sind, um den Löchern (1b, 1c) des proximalen Teils (1a) des Nagels (1) zu entsprechen.
